# FASCICULE DE BREVET EUROPEEN

(11) **EP 0 698 595 B1**
(45) Date de publication et mention de la délivrance du brevet: **24.06.1998**
(21) Numéro de dépôt: 95401822.2
(22) Date de dépôt: 03.08.1995
(51) Int. Cl.: C07C 67/14, C07C 69/017, A61K 7/00, A61K 31/23

(54) **Procédé d'estérification d'un extrait oligomère polyphénolique d'origine végétale, composition ainsi obtenue et son utilisation**
Veresterungsverfahren eines oligomeren polyphenolischen Extraktes pflanzlicher Herkunft, die so erhaltene Zusammensetzung und ihre Anwendung
Process for esterifying an oligomeric polyphenolic phytogenic extract, the obtained composition and its use

(30) Priorité: 26.08.1994 FR 9410317
(43) Date de publication de la demande: 28.02.1996
(73) Titulaire: BERKEM, 24680 Gardonne (FR)
(72) Inventeur: Nkiliza, Jean, I-33220 Port Sainte Foy (FR)
(74) Mandataire: Peuscet, Jacques

(56) Documents cités:
- EP-A- 0 252 724
- DE-A- 1 543 537

## Description

L'invention concerne un procédé d'estérification d'extrait oligomère polyphénolique d'origine végétale, notamment pour obtenir des esters d'acide gras saturés.

Les composés polyphénoliques peu polymérisés ou non polymérisés d'origine végétale, notamment les acides hydroxycinnamiques, les flavonoïdes et les flavanols, possèdent des propriétés biologiques intéressantes parmi lesquelles on peut citer, à titre d'exemple, un effet capteur de radicaux libres, un effet antioxydant, un effet antimicrobien et antiviral, un effet de filtre solaire naturel.

Leurs fonctions phénoliques rendent ces composés particulièrement sensibles aux différents facteurs de dégradation tels que la lumière, l'air et les pH acide ou basique. Cette instabilité constitue, dans la majorité des cas, un frein à l'exploitation de leurs effets bénéfiques tant pour l'homme que pour les animaux. L'importance pharmacologique de ces polyphénols rend particulièrement souhaitable la mise au point d'un moyen susceptible de leur conférer une bonne stabilité compatible avec leurs applications biologiques.

Dans l'état de la technique, on a décrit, à des fins de détermination structurale par des méthodes de résonance magnétique nucléaire, l'obtention de produits individuels purs par chromatographie sur colonne de silice ou sur couche mince après un traitement ayant pour but de protéger les fonctions phénoliques libres qui, sans cette précaution, seraient considérablement dégradées par le support. Le traitement en cause consiste à protéger les fonctions phénoliques par éthérification ou estérification et améliore considérablement la stabilité. On obtient ainsi des produits, qui sont solubles dans les solvants organiques peu polaires, tels que le chloroforme ou le chlorure de méthylène. Les éthers les plus utilisés sont les éthers méthyliques obtenus en traitant les solutions phénoliques soit avec du diméthyl sulfate, soit avec du diazométhane. Les esters les plus utilisés sont des peracétates préparés par action d'un mélange de pyridine et d'anhydride acétique à l'abri de la lumière, à température ambiante pendant au moins 24 heures ou à environ 80°C pendant environ 3 heures. Ce procédé est, par exemple, décrit dans J.B. HARBONE "Methods in plant biochemistry. Volume I : Plant phenolics", page 23, Academic press, 1989.

L'invention a pour but de proposer un procédé économique de préparation de polyphénols stabilisés par estérification, ces produits étant destinés à l'industrie pharmaceutique, cosmétique et alimentaire. Selon l'invention, les dérivés que l'on prépare ont une grande liposolubilité les rendant compatibles avec les excipients des formes galléniques et permettent, en outre, une régénération aisée des phénols libres, qui sont à la base des propriétés recherchées par l'administration du produit.

Pour atteindre les objectifs ci-dessus définis, on a proposé, selon l'invention, d'estérifier les extraits polyphénoliques par des dérivés d'acides gras à longues chaînes hydrocarbonées. Les esters obtenus ont un aspect comparable à celui des triglycérides et sont solubles dans les solvants des graisses. Après administration, ces esters peuvent subir une hydrolyse enzymatique, comme les lipides, pour régénérer l'acide gras et les polyphénols natifs. Pour éviter des effets secondaires indésirables, on propose selon l'invention d'utiliser des dérivés des acides gras les plus répandus dans les corps gras employés en alimentation humaine, notamment l'acide palmitique et l'acide stéarique ; pour éviter les phénomènes d'oxydation, on considèrera, de préférence, des dérivés d'acides gras saturés.

On sait que l'estérification d'un phénol peut, de façon connue, se faire selon plusieurs voies :
a) on peut réaliser une réaction directe entre le composé hydroxylé et l'acide carboxylique, à haute température sous vide ou par catalyse avec un acide minéral, en éliminant, par entraînement azéotropique, l'eau formée ; mais une telle technique est incompatible avec la faible stabilité et le comportement chimique des polyphénols considérés.
b) on peut réaliser une condensation directe entre le composé hydroxylé et un acide carboxylique en utilisant un agent de couplage et un catalyseur basique de faible nucléophilie, l'agent de couplage couramment utilisé étant la dicyclohexylcarbodiimide, qui active l'acide carboxylique en le transformant en un équivalent d'anhydride et la base utilisée étant généralement la 4-diméthylaminopyridine ; mais cette technique présente deux inconvénients économiques majeurs :
   - en premier lieu, elle est d'un coût élevé en raison du prix important de la dicyclohexylcarbodiimide, qui est utilisée en quantité stoechiométrique, voir en léger excès, par rapport à l'acide carboxylique,
   - la dicyclohexylcarbodiimide se transforme en dicyclohexylurée insoluble dans le milieu réactionnel et produite en quantité équivalente à la dicyclohexylcarbodiimide mise en oeuvre. Ce sous-produit doit être considéré comme un déchet du procédé et sa destruction augmente encore le coût de production des esters obtenus par cette méthode.
c) on peut faire réagir le composé hydroxylé avec un dérivé fortement réactif de l'acide carboxylique, par exemple un chlorure ou un anhydride d'acide, en présence d'une base. La mise en oeuvre de cette modalité réactionnelle dépend surtout du choix de la base, qui joue à la fois le rôle de catalyseur et de piège de l'acide minéral formé (HCl dans le cas d'un chlorure d'acyle) ; la réaction peut s'effectuer en solution aqueuse alcaline selon la procédure de SCHOTTEN-BAUMANN. Dans l'état de la technique, on propose généralement d'utiliser la pyridine comme catalyseur, la pyridine servant à la fois de solvant de réaction, de base et de piège de l'acide libéré par la réaction. Cependant, à l'échelle industrielle, l'usage de la pyridine présente des inconvénients considérables : en premier lieu, la pyridine est toxique pour l'homme et elle est susceptible de pénétrer dans l'organisme par inhalation, par voie cutanée ou par ingestion en provoquant des effets dépresseurs, des troubles hépatiques et rénaux ou des irritations de la peau et des muqueuses ; en deuxième lieu, la pyridine a une odeur désagréable et pénétrante, ce qui exige une purification parfaite du produit final par des lavages multiples, notamment à l'eau acidulée ou avec une solution contenant des ions cuivriques: or, dans le cas des esters gras de polyphénols, ces lavages répétés entraînent une formation d'émulsions relativement stables, ce qui baisse fortement le rendement de la production et augmente, en conséquence, le prix de revient des esters correspondants.

Il résulte de cette analyse des techniques, qui sont à la disposition de l'homme de métier et qui ont été ci-dessus résumées, qu'aucune de ces techniques n'est à ce jour, susceptible d'être utilisée pour une production économique d'esters d'acide gras d'oligomères polyphénoliques.

La présente invention a, en conséquence, pour objet un procédé d'estérification d'un extrait oligomère polyphénolique d'origine végétale par action d'un chlorure d'acide, caractérisé par le fait que l'on place ledit extrait dans un milieu liquide non solvant pour ledit extrait mais solvant pour le(s) ester(s) à obtenir, de façon à obtenir une suspension ; que l'on ajoute à ladite suspension au moins une amine tertiaire aliphatique à bas point d'ébullition en présence d'une quantité catalytique d'au moins une base organique autre que la pyridine ; et que l'on introduit dans ce mélange au moins un chlorure d'acide gras, le mélange réactionnel étant agité à une température inférieure à 40°C puis concentré par évaporation pour obtenir un extrait estérifié.

Dans un mode préféré de mise en oeuvre du procédé selon l'invention, on choisit comme amine tertiaire aliphatique la triéthylamine ; on peut également choisir comme base organique ajoutée en quantité catalytique la 4-diméthylaminopyridine.

Avantageusement, on peut choisir comme solvant de réaction un solvant chloré, tel que, par exemple, le chloroforme ou le chlorure de méthylène.

Le chlorure d'acide que l'on met en oeuvre est avantageusement un chlorure d'acide gras saturé présent dans les corps gras naturels, notamment un chlorure choisi dans le groupe formé par les chlorures d'acide palmitique, stéarique et laurique.

On constate que le procédé selon l'invention est extrêmement économique étant donné que le coût de la triéthylamine est de loin inférieur à celui de la pyridine ou de la dicyclohexylcarbodiimide. Par ailleurs, la mise en oeuvre de la triéthylamine présente beaucoup moins de risque que la mise en oeuvre de la pyridine, car elle est facilement éliminée par évaporation et les traces résiduelles sont entraînées par lavage du produit obtenu avec un solvant polaire tel que le méthanol, l'éthanol, l'acétone, l'eau ou leurs mélanges, par exemple. La 4-diméthylaminopyridine utilisée en quantité catalytique comme base est éliminée lors des lavages du fait de sa grande solubilité dans les solvants de lavage.

La dernière étape du procédé selon l'invention est une concentration permettant d'obtenir l'extrait estérifié. Cette concentration peut, avantageusement, être effectuée par évaporation à sec sous pression réduite à une température inférieure à 40°C ; le produit sec ainsi obtenu peut être purifié par lavage, de préférence par plusieurs lavages successifs, avec au moins un solvant polaire tel que le méthanol, l'éthanol, l'acétone, l'eau ou leurs mélanges. Après un tel lavage, le produit est séché à une température inférieure à 40°C, ce séchage pouvant être réalisé soit à l'air libre, soit sous vide, soit dans une étuve ventilée.

Compte tenu de leur hydrolyse enzymatique dans l'organisme, les polyphénols estérifiés ainsi obtenus conservent les applications habituelles des extraits polyphénoliques à l'état natif ; ils peuvent être administrés en cosmétique par voie topique ou en thérapie humaine par voie topique ou administration orale.

Pour mieux faire comprendre l'objet de l'invention, on va en décrire maintenant, à titre d'exemple purement illustratif et non limitatif, un mode de mise en oeuvre.

### EXEMPLE

On réalise une suspension de 100 grammes d'oligomères polyphénoliques extraits des pépins de raisin dans 600 ml de chloroforme. On ajoute à cette suspension 250 ml de triéthylamine et une quantité catalytique de 7 grammes de 4-diméthylaminopyridine. On agite ce mélange mécaniquement sous azote et on introduit lentement 400 ml de chlorure de palmitoyle. Lorsque l'addition est terminée, on agite à température ambiante pendant 12 heures. Après cette période de réaction, on évapore à sec sous pression réduite en maintenant la température au-dessous de 40°C.

On reprend ensuite le résidu avec 1 litre d'un mélange méthanol/eau (9/1). On agite le mélange pendant 1 heure à température ambiante, on écarte le surnageant, on ajoute ensuite 1 litre de méthanol et on poursuit l'agitation à température ambiante pendant 1 heure. On filtre ce mélange et on lave le solide avec de l'acétone puis on le sèche.

Le produit obtenu après séchage se présente sous forme d'une poudre de consistance grasse, insoluble dans les solvants polaires et solubles dans les solvants des graisses, tels que l'hexane ou les solvants chlorés par exemple. Le spectre infrarouge, réalisé sur un film laissé par évaporation du chloroforme sur une pastille de NaCl, signale les fonctions ester par une bande d'absorption à 1 725 cm⁻¹ ; l'absence de bande d'absorption entre 3 600 et 3 100 cm⁻¹ témoigne de l'absence de groupes hydroxyles libres. Le spectre ultraviolet présente un maximum d'absorption autour de 273 nanomètres.

## Revendications

1. Procédé d'estérification d'un extrait oligomère polyphénolique d'origine végétale par action d'un chlorure d'acide, caractérisé par le fait que l'on place ledit extrait dans un milieu liquide non solvant pour ledit extrait mais solvant pour le(s) ester(s) à obtenir, de façon à obtenir une suspension ; que l'on ajoute à ladite suspension au moins une amine tertiaire aliphatique à bas point d'ébullition en présence d'une quantité catalytique d'au moins une base organique autre que la pyridine ; et que l'on introduit dans ce mélange au moins un chlorure d'acide gras, le mélange réactionnel étant agité à une température inférieure à 40°C puis concentré par évaporation pour obtenir un extrait estérifié.

2. Procédé selon la revendication 1, caractérisé par le fait que l'on choisit comme amine tertiaire aliphatique, la triéthylamine.

3. Procédé selon l'une des revendications 1 ou 2, caractérisé par le fait que l'on choisit comme base organique ajoutée en quantité catalytique, la 4-diméthylaminopyridine.

4. Procédé selon l'une des revendications 1 à 3, caractérisé par le fait que l'on choisit comme solvant de réaction, un solvant chloré.

5. Procédé selon la revendication 4, caractérisé par le fait que l'on choisit le solvant chloré dans le groupe formé par le chloroforme et le chlorure de méthylène.

6. Procédé selon l'une des revendications 1 à 5, caractérisé par le fait que l'on choisit le chlorure d'acide dans le groupe formé par les chlorures d'acides gras saturés présents dans les corps gras naturels.

7. Procédé selon la revendication 6, caractérisé par le fait que l'on choisit le chlorure d'acide dans le groupe formé par les chlorures d'acide palmitique, stéarique et laurique.

8. Procédé selon l'une des revendications 1 à 7, caractérisé par le fait que l'on effectue la concentration de l'extrait estérifié par évaporation à sec sous pression réduite à une température inférieure à 40°C.

9. Procédé selon l'une des revendications 1 à 8, caractérisé par le fait que l'on purifie l'extrait estérifié obtenu après concentration, par lavage(s) avec au moins un solvant polaire, puis séchage à une température inférieure à 40 °C.

10. Composition d'oligomères polyphénoliques estérifiés susceptible d'être obtenue par le procédé selon l'une des revendications 1 à 9.

11. Utilisation d'une composition selon la revendication 10 en cosmétique par voie topique ou administration orale.

12. Composition pharmaceutique contenant une composition selon la revendication 10 pour application par voie topique ou orale.

## Claims

1. Process for esterifying a polyphenolic oligomer extract of plant origin by the action of an acid chloride, characterized in that the said extract is placed in a liquid medium which is not a solvent for the said extract but which is a solvent for the ester(s) to be obtained, so as to obtain a suspension; in that at least one aliphatic tertiary amine of low boiling point is added to the said suspension in the presence of a catalytic amount of at least one organic base other than pyridine; and in that at least one fatty acid chloride is introduced into this mixture, the reaction mixture being stirred at a temperature below 40 °C and then concentrated by evaporation in order to obtain an esterified extract.

2. Process according to Claim 1, characterized in that triethylamine is chosen as the aliphatic tertiary amine.

3. Process according to either of Claims 1 and 2, characterized in that 4-dimethylaminopyridine is chosen as the organic base added in catalytic amount.

4. Process according to one of claims 1 to 3, characterized in that a chlorinated solvent is chosen as the reaction solvent.

5. Process according to Claim 4, characterized in that the chlorinated solvent is chosen from the group formed by chloroform end methylene chloride.

6. Process according to one of Claims 1 to 5, characterized in that the acid chloride is chosen from the group formed by the chlorides of saturated fatty acids present in natural fats.

7. Process according to Claim 6, characterized in that the acid chloride is chosen from the group formed by palmitoyl chloride, stearoyl chloride and lauroyl chloride.

8. Process according to one of Claims 1 to 7, characterized in that concentration of the esterified extract is carried out by evaporation to dryness under reduced pressure at a temperature below 40°C.

9. Process according to one of Claims 1 to 8, characterized in that the esterified extract obtained after concentration is purified by washing (possibly more than once) with at least one polar solvent, followed by drying at a temperature below 40°C.

10. Composition of esterified polyphenolic oligomers which is capable of being obtained by the process according to one of Claims 1 to 9.

11. Topical or oral use in cosmetics of a composition according to Claim 10.

12. Pharmaceutical composition containing a composition according to Claim 10 for topical or oral application.

## Patentansprüche

1. Verfahren zur Veresterung eines Extraktes oligomerer Polyphenole pflanzlichen Ursprungs mit einem Säurechlorid, dadurch gekennzeichnet, daß man den Extrakt in ein flüssiges Medium gibt, welches zwar nicht den Extrakt, jedoch den (die) gewünschten Ester löst, so daß man eine Suspension erhält; zu der Suspension wenigstens ein aliphatisches tertiäres Amin mit niedrigem Siedepunkt in Gegenwart einer katalytischen Menge wenigstens einer von Pyridin verschiedenen organischen Base gibt; und in dieses Gemisch wenigstens ein Fettsäurechlorid einbringt, wobei das Reaktionsgemisch bei einer Temperatur von weniger als 40°C gerührt und dann durch verdampfen eingeengt wird, um einen veresterten Extrakt zu erhalten.

2. Verfahren nach Anspruch 1, dadurch gekennzeichnet, daß man Triethylamin als aliphatisches tertiäres Amin wählt.

3. Verfahren nach einem der Ansprüche 1 oder 2, dadurch gekennzeichnet, daß man 4-Dimethylaminopyridin als organische Base wählt, die in katalytischen Mengen zugegeben wird.

4. Verfahren nach einem der Ansprüche 1 bis 3, dadurch gekennzeichnet, daß man ein chloriertes Lösungsmittel als Reaktionslösungsmittel wählt.

5. Verfahren nach Anspruch 4, dadurch gekennzeichnet, daß man das chlorierte Lösungsmittel auswählt unter Chloroform und Methylenchlorid.

6. Verfahren nach einem der Ansprüche 1 bis 5, dadurch gekennzeichnet, daß man das Säurechlorid auswählt unter gesättigten Fettsäurechloriden, die in natürlichen Fettkörpern vorhanden sind.

7. Verfahren nach Anspruch 6, dadurch gekennzeichnet, daß man das Säurechlorid auswählt unter Palmitin-, Stearin- und Laurinsäurechlorid.

8. Verfahren nach einem der Ansprüche 1 bis 7, dadurch gekennzeichnet, daß man den veresterten Extrakt einengt, indem man unter vermindertem Druck bei einer Temperatur von weniger als 40°C zur Trockne eindampft.

9. Verfahren nach einem der Ansprüche 1 bis 8, dadurch gekennzeichnet, daß man den eingeengten veresterten Extrakt reinigt, indem man ihn mit wenigstens einem polaren Lösungsmittel wäscht und dann bei einer Temperatur von weniger als 40°C trocknet.

10. Zusammensetzung aus veresterten oligomeren Polyphenolen, die durch ein verfahren nach einem der Ansprüche 1 bis 9 erhältlich sind.

11. Verwendung einer Zusammensetzung nach Anspruch 10 in der Kosmetik auf topischem Weg oder durch orale verabreichung.

12. Pharmazeutisches Mittel, enthaltend eine Zusammensetzung nach Anspruch 10 zur topischen oder oralen Anwendung.
